# EUROPEAN PATENT APPLICATION

(11) **EP 4 763 252 A1**
(43) Date of publication of application: **24.06.2026**
(21) Application number: 24221045.8
(22) Date of filing: 18.12.2024
(51) Int. Cl.: A61M 5/46, A61M 5/158

(54) **IMPROVED INFUSION SET**

(71) Applicant: mylife Diabetes Care AG, 3400 Burgdorf (CH)
(72) Inventor: STUCKI, Janick, 3400 Burgdorf (CH); ZIEMENDORFF, Etienne, 8050 Zürich (CH); MÖRI, Nadia, 4542 Luterbach (CH)
(74) Representative: Finklenburg, Susanne

(57) **Abstract**

An infusion set comprising a base having upper and lower sides. The base comprises a moveable infusion cannula which allows for an insertion depth adjustment of the infusion cannula by a using person prior to insertion of the infusion cannula into tissue.

## Description

### FIELD OF THE INVENTION

The present invention relates to the subcutaneous infusion of fluid medicament. In particular, the invention relates to infusion sets for subcutaneous infusion of fluid medicament.

### BACKGROUND OF THE INVENTION

Infusion sets for subcutaneous infusion have been known for decades. Such infusion sets are usually releasably connected to an infusion pump by a connector fixed to one end of the infusion set. A flexible tubing is arranged between the connector and a connector cannula. The connector cannula is releasably attached to a patch device comprising the actual infusion cannula, which may be inserted into the subcutaneous tissue of a using person. A fluid path is formed between the connector and the infusion cannula through the tubing and the connector cannula.

The patch device usually comprises a base with a lower and an upper side. On the lower side a patch is arranged to be attached to the user's skin. Extending from the lower side is the infusion cannula. On the upper side an attachment mechanism for the connector cannula is arranged. The base comprises a cannula hub on the upper side which reaches through the base to the cannula. The cannula hub acts as connection piece between the connector cannula and the infusion cannula. When the connection cannula is connected to the patch device, connection cannula is inserted into a lumen of the cannula therefore forming a fluid connection with the infusion cannula. In many cases the end of the cannula hub directed away from the infusion cannula is closed by a septum which is pierceable.

In US7309326 B2 the infusion cannula is fixedly attached the base of the patch device. It may be made from a soft material like a polymer, e. g. like PTFE, or from a stiff material like steel. As the infusion cannula is fixed also the length of the cannula potentially reaching into the tissue of the using person is pre-determined at the time of manufacture. Hence to accommodate different bodies of using people, the infusion sets have to be produced in many different variations, with varying lengths of the infusion cannula. Depending on the location where the infusion set is applied to the body, the using people have to keep different variations in stock as well.

There is the need for improved infusion sets which allow for reduced numbers of variations.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

### DESCRIPTION OF THE INVENTION

It is an objective of the invention to provide adaptable infusion sets which reduce the burden of producing and keeping in stock many variations of the infusion sets with varying infusion cannula lengths. It is a further objective of the invention to provide an infusion set to users which allows them flexibly vary the desired insertion length of the infusion cannula.

This objectives are achieved by the technical teaching of claim 1. Advantageous advancements are described in the dependent claims, the description and the figures.

In one aspect, an infusion set according to the invention comprises a base which has a lower side and an upper side. Disposed fixedly attached to the lower side of the base is a flexible and adhesive patch. The patch is configured to be attached to the skin of a using person. The base comprises an opening between the upper side and the lower side. The actual infusion cannula has a tip (distal end) extending from the lower side of the base and an opposite end with a hub, wherein the hub constitutes part of the proximal end of the infusion cannula. The infusion cannula has a longitudinal axis. The hub has a diameter bigger than the actual infusion cannula and is disposed moveably in the opening of the base. The hub has a fluid passage which allows fluid to pass from an open end of the hub into the lumen of the infusion cannula. The hub has a roughly cylindrical shape with the fluid passage as directed along the axis as a lumen. A thread is arranged on an outer lateral surface of the hub. Correspondingly a mating threaded inner surface is arranged on an inner surface of the opening. The hub is therefore moveable along the threaded connection. The hub may be moveable between upper end position and a lower end position, each provided in the opening. The infusion set further comprises a protective sleeve removably (but rotationally fixed) attached to the hub. The protective sleeve covers the infusion cannula prior to insertion into tissue of a using person and is removed from the infusion set (detached from the hub) immediately before insertion. The protective sleeve covers and surrounds the infusion cannula up the hub of the infusion cannula. When attached to the hub, rotation of the protective sleeve around its longitudinal axis is transferred to the hub, the hub hence rotating as well and moving along the thread. This allows an optimal setting of the insertion depth for the infusion cannula without having the risk of inadvertently touching the infusion cannula as it is protected by the protection sleeve.

In one aspect the base may have a circular shape, i- e. a disc-like shape.

In one aspect the described threaded inner surface of the opening comprises elements which limit the movement of the hub, these elements have the shape of stop surfaces or end of thread blocks. Typically, there is at least one element limiting movement of the hub on the lower side of the base and at least one element limiting movement on the upper side of the base. In one possible configuration the elements may stop movement in axial direction of the threaded connection. In another possible configuration the elements may stop rotational movement of the hub. In yet another possible configuration the elements stop both, the rotational movement and the axial movement of the hub. In the first case the elements may comprise stop surfaces which are oriented approximately perpendicular to the longitudinal axis of the infusion cannula (and the rotational axis of the hub). The expression used for this kind of stop surface is axial stop surface or simply axial stop. In the second case the elements may comprise stop surfaces oriented roughly parallel to the longitudinal axis of the infusion cannula. The expression used for this kind of stop surface is radial stop surface or simply radial stop surface. For the third described possible configuration of elements the axial and the radial stops are combined.

As described above, the hub has an inner lumen. And this inner lumen has the function to provide a fluid connection or fluid passage into the lumen of the infusion cannula. In one aspect, the inner lumen of the hub is configured to comprise an elastic and pierceable septum which closes the access to the fluid connection with infusion cannula. The septum may therefore serve as protection of the lumen and of the lumen of the infusion cannula. In one case, the septum is a lid-like structure for the hub on the proximal end of the hub.

The inner lumen of the hub may have a cylindrical shape. Alternatively, the shape of the inner lumen transitions in distal direction from cylindrical into a funnel-like shape with a distal opening having approximately the same diameter as the diameter of the lumen of the infusion cannula. In a further alternative arrangement, the overall shape of the inner lumen of the hub may be funnel-like, with a small opening on its distal end.

In one aspect the infusion set as described in its variants above and below further comprises an infusion cap. The infusion cap is configured to be removably attached to the upper side of the base. The cap may be rotatably attached to the to the base, such that the infusion cap may rotate on the base when being attached to the base. Rotation is advantageously around the longitudinal axis of the infusion cannula possible. The infusion cap comprises a cap cannula or connection cannula extending from a lower side of the infusion cap, the cap cannula is configured to pierce the septum and to be introduced into the lumen of the hub, through the septum if a septum is present. If the septum is present it is pierced (by the tip of the cap cannula).

In one aspect the infusion cap is fixedly connected to an infusion tubing which is in fluid connection with the lumen of the cap cannula. When such an infusion cap is connected to the base, fluid medicament may be infused from the infusion tubing through the cap cannula into the infusion cannula and subsequently into the tissue of the using person.

In one aspect the infusion cannula is made from soft material, like a polymeric material. In this aspect the infusion set according to the invention further comprises an introducer cap. The introducer cap comprises an introducer needle, which extends from a lower side of the introducer cap. The introducer cap is adapted to be removably attached to the base such that the introducer needle is inserted into the infusion cannula and extends beyond the tip of the infusion cannula. Hence, if a septum is present in the hub of the infusion cannula, the introducer needle pierces the septum to reach through the infusion cannula. The introducer needle may be fixedly and non-movably attached to the introducer cap, or the introducer needle may be arranged in a moveable manner to the introducer cap.

In an advancement of the just described aspect, the introducer cap may comprise at least one retaining wing and at least one retaining tab, both arranged on an upper side of the introducer cap.

The retaining wing(s) and retaining tab(s) are firstly configured to be grabbed by a using person for manual insertion of the infusion cannula into the tissue of the using person, and secondly are configured to be used with an insertion device, wherein the at least one retaining wing and the at the least one retaining tab may be configured to be releasably retained by the insertion device. An insertion device as could be used for the present invention is described in detail in US10335543 B2, which hereby is fully incorporated by reference.

As described, the introducer needle may be attached to the introducer cap, such that the needle is not moveable relative to the introducer cap. In such cases the infusion cannula moves along the longitudinal axis of the introducer needle when setting the length of the infusion cannula extending from the lower side of the base.

In an alternative, the introducer needle is arranged moveably on the lower side of the introducer cap. For this arrangement, a threaded sleeve is fixed to the lower side of the inserter cap. The threaded sleeve is disposed in the center of the introducer cap, such that when the introducer cap is attached to the base, the threaded inner surface of the base and the threaded sleeve of the introducer cap are arranged co-axially. Furthermore, and preferably the threads of the threaded inner surface and the threaded sleeve have the same pitch. So, one full rotation is related to the identical axial displacement. The end of the insertion needle pointing to the introducer cap comprises a thread, which mates the thread of the threaded sleeve. The thread comprising end of the introducer needle may comprise a cylinder-like structure with a diameter bigger than the diameter of the introducer needle. The cylinder-like structure comprises the thread mating the thread of the threaded sleeve. The threaded connection between the introducer needle and the threaded sleeve has the same pitch as the threaded connection between the infusion cannula hub and the base. So, when the introducer cap is attached to the base and the protective sleeve is rotated, the introducer needle may rotated in unison with the infusion cannula. The hub of the infusion cannula and the cylindrical structure of the introducer needle may each comprise one or more coupling elements, which rotationally couple the introducer needle and the infusion cannula when the introducer cap is attached to the base. The coupling elements may of mating male/female hexagonal shapes like with hexagonal wrenches and screws. Alternatively, the coupling shape may be rectangular, triangular or have a star-like shape (like Torx^{™}) - which can improve torque transfer.

In one aspect of the invention the infusion cannula is made from a biocompatible metal or metal alloy. For example, the infusion cannula may be made from a steel of type AISI 301, 304, 316, or similar. Alternatively, the cannula may be made from commercially pure titanium or a titanium alloy. In further alternative, the cannula may be made from a biocompatible and/or biosoluble/bioresorbable magnesium-based material.

In a further aspect of the invention the threaded connection between the hub and opening of the basis further comprises a two-way ratchet connection such that the angular position and therefore, the axial position of the infusion cannula may have (meta-)stable states. For example, the threaded outer surface of the hub may comprise one or multiple ratchet teeth on the ridges of the thread and correspondingly, the valley of the thread on the threaded inner surface of the opening in the base may comprise flexible elements interacting with the teeth, therefore forming a ratchet, the ratchet allowing for bi-directional movement. In one variant the teeth may have a symmetrical shape such that the resistance for the flexible elements to flex over the teeth is the same in both directions of rotation. In another variant the teeth may have asymmetrical shape to have different resistances in the two directions of rotation. In a further variant it is possible to have teeth on the thread ridges of the opening in the base and the flexible elements on the hub of the infusion cannula. In one possibility the flexible elements may be formed from an arm which is on one end fixedly attached to the thread surface and on the other end is free to flex and comprises a tooth disposed on the free end which interacts with the teeth on the thread ridges.

In one aspect of the invention relates to the distance which the hub may travel between the stop surfaces at lower and the upper end of the threaded connection respectively. Typically, soft and steel cannulas have an insertion depth between 5 and 9 mm. Accordingly, the travel distance would ideally cover 4 mm, such that a change from 9 to 5 mm insertion depth would be possible. However, in certain cases a travel distance of less than 4 mm may be more preferable, as the possible travel distance has an impact on the overall thickness of the base. However, the minimal travel distance should be 2 mm or more. In a development of the present aspect, e.g. for children, specific smaller insertion depth may be useful. I. e. a range of 3 to 6 mm.

In one aspect of the invention the pitch of the threaded connection is chosen, such that the threaded connection is self-locking which means that purely axial force applied to the protective sleeve does not induce rotation of the hub. Which is the case when the thread angle of the thread is smaller than the friction angle between the contacting surfaces of the thread. Details may be obtained from any mechanics text book and are known to a person skilled in the art.

In one aspect of the invention the protective sleeve comprises markings on the outside of sleeve, marking pre-determined insertion depth for the infusion cannula. The markings may be color markings or tactile markings or combinations thereof. The markings may be applied as one or more rings on the surface of the protective sleeve. For example, one colored and tactile ring which may be only visible and/or palpable when the set depth is 9mm and one ring which disappears when the minimal insertion depth is reached.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts the infusion set base 1 with introducer cap 5 attached to the base (also shown: protective sleeve 6);
- Fig. 2: depicts an exploded view of figure 1 with shown infusion cannula 2, septum 4 and hub 3;
- Fig. 3a: depicts a longitudinal cross-section through the setup of figure 1 where the hub of the infusion cannula 3 and the infusion cannula are in their lowest possible position, meaning maximum insertion depth;
- Fig. 3b: depicts a longitudinal cross-section through the setup of figure 1 where the hub of the infusion cannula 3 and the infusion cannula are in their uppermost possible position, meaning minimum insertion depth;
- Fig. 4: depicts an alternative infusion set base 10 with an introducer cap 15 and protective sleeve 16;
- Fig. 5: depicts a longitudinal cross-section of figure 4 showing the soft infusion cannula 12, the hub 13 with the septum 14, the introducer needle 15a, the threaded sleeve 15b of the introducer cap 15 and the hub 15c of the introducer needle 15a;
- Fig. 6: depicts fully exploded view of figure 4 (septum 14 in hub 13 not shown);
- Fig. 7a: depicts a partly exploded view of figure 4 with a focus on the kinematic coupling between the 13 and the hub 15c which found in a magnified view in
- Fig. 7b,: where the rotational connection between coupling end 13c and coupling 15d is shown;
- Fig. 8: depicts infusion set base 1 with the infusion cap 27 attached to it, where the connection cannula 27a of the infusion cap 27 pierces septum 4 located in hub 3.
- Fig. 9a: depict alternative base 20 with the radial stops 20g and 20f as shown in detail views in
- Fig. 9b: and
- Fig. 9c,: respectively;
- Fig. 10a: depicts alternative base 30 with axial stops 30f and 30g as shown in detail views in
- Fig. 10b: and
- Fig. 10c,: respectively;
- Fig. 11a: depicts alternative base 40 with infusion cannula hub 43 where the hub 43 is positioned most to the right side on the drawing indication minimal insertion depth;
- Fig. 11b: depicts a view onto base 40 onto the lower side of the base 40 with liner 40h of the adhesive patch 40c (not visible), where the coupling groove 43 for the protective sleeve (not shown, but identical to protective sleeve 6) is visible and where the markers 40k are arranged on the liner 40h;
- Fig. 11c: depicts the detail H of figure 11b, where the insertion depth marker 40k for 6 mm insertion depth is aligned with the marking 43c of the hub 43;
- Figs. 12a -12c: depict the same base 40 and parts as in figures 11a to 11c but with the hub 43 positioned for maximum insertion depth of the infusion cannula;
- Fig. 13a: depicts an exploded view onto the base 40 and the hub 43 as shown in figures 12a-12c;
- Fig. 13b: depicts a rotated exploded view of the arrangement shown in figure 13a.

The reference symbols used in the drawings, and their primary meanings, are listed in summary form in the list of designations. Analog parts are provided with the same reference symbols in the figures but with increased numbering.

### DETAILED DESCRIPTION OF PREFERRED EMBODIMENTS

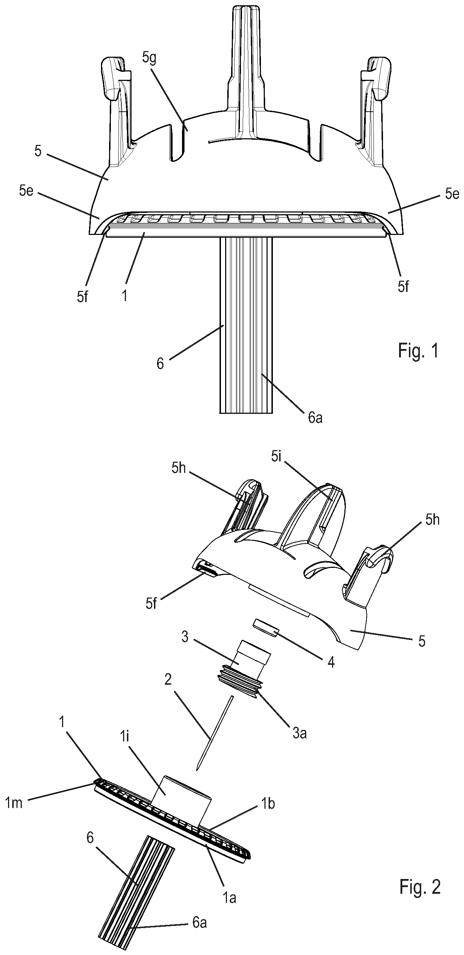

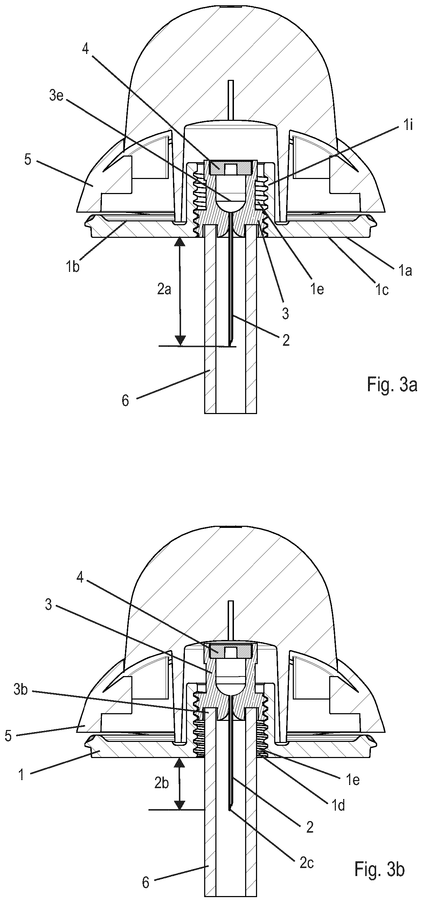

A first embodiment of the invention is shown in figures 1 to 3b. Figure 1 shows the infusion set base 1 with the introducer cap 5 attached to the base 1 by a snap-on connection. The base 1 has a circular main shape with a rim 1m arranged on the circumference of base 1. Introducer cap 5 is attached to the base by the teeth 5f being snapped over the rim 1m. For this that section of the introducer cap 5 near teeth 5f comprises the flexible area 5e. In order to increase the flexibility of the introducer cap cuts 5g may be arranged on the cap 5. Furthermore, the introducer cap 5 in the shown example comprises two retaining wings 5h and a retaining tab 5i which are configured to be releasably inserted in and attached to an infusion set insertion device (not shown). The infusion set insertion device is configured to automatically insert the infusion cannula into the tissue of a patient. An example insertion device is disclosed in US10335543 B2, which is incorporated by reference in an above section. The retaining wings 5h are also used as gripping element during manual insertion. The base 1 has a lower side 1a and an upper side 1b (ref. figure 2). As can be seen in figure 1, the protective sleeve 6 is mounted to the hub 3 (see figure 3a) and comprises longitudinal ribs 6a, the longitudinal ribs 6a are configured as gripping elements. Protective sleeve 6 is detachably mounted to hub 3 but rotationally fixed, such that a using person may grip the longitudinal ribs 6a to rotate protecting sleeve 6 and thereby also rotating hub 3. Figure 2 shows more details of the first embodiment, namely the infusion cannula 2 and the infusion cannula hub 3 with its outer thread 3a and the septum 4. Base 1 also comprises a cylindrical wall 1i extending from the upper side 1b of the base 1. Figure 3a and 3b show longitudinal cross-sections through the arrangement of figure 1. The infusion cannula is fixedly and non-moveably attached to the infusion cannula hub 3. Inserted into the upper opening of the hub 3 is the septum closing the lumen 3e. As already shown in figure 2 the hub 3 comprises a thread 3a on its outer surface. The base has an opening 1d to which the wall 1i is aligned. On its inner surface opening 1d and wall 1i comprise the thread 1e which matches the thread 3a of the hub 3. Hub 3 may therefore be moved along the threading between an upper end position as shown in figure 3b and a lower end position as shown in figure 3a. By moving the hub 3 along the threading 3a/1e also the infusion cannula 2 is moved, hence, changing the insertion depth between 2a and 2b. As already mentioned in the general description, the insertion depth may vary between about 3 and about 10 mm. The infusion cannula is made from stiff material like steel such that no introducer needle is needed to help insertion of the infusion cannula into tissue of a using person.

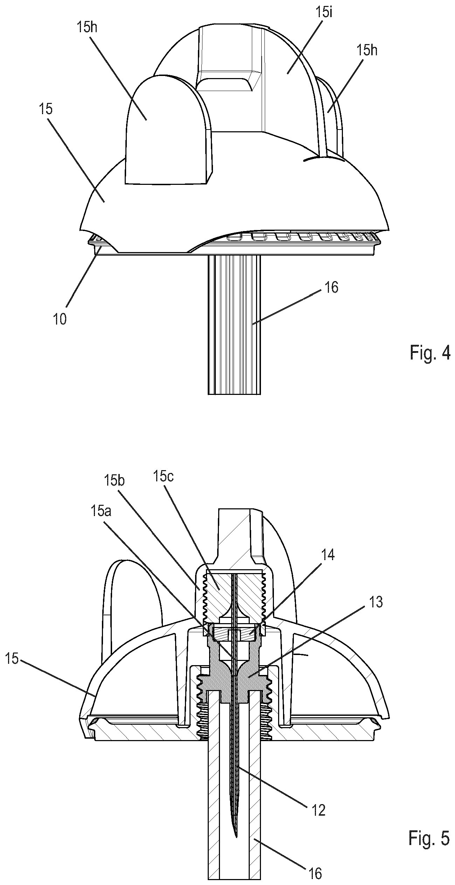

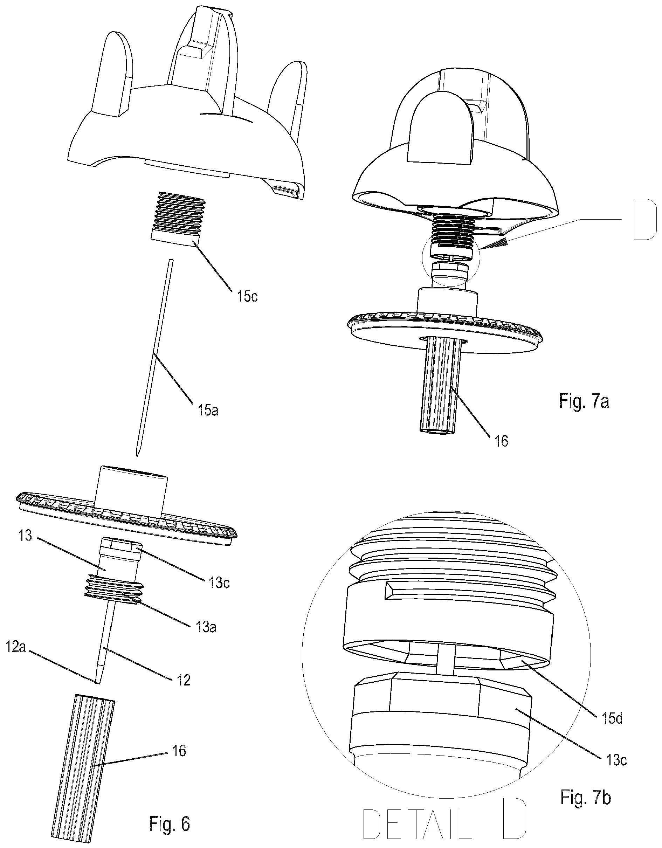

A second embodiment of the invention is shown in figures 4 to 7b. The difference between the first and the second embodiment is mainly that the infusion cannula 12 is made from a soft material like PTFE (also known as Teflon^{™}). This change has a number of consequences for the design of the infusion set. Firstly, to insert the soft infusion cannula 12 into tissue an introducer needle 15a is needed to guide insertion. After insertion the introducer needle 15a is removed together with the introducer cap 15. As shown in the figures 5 to 7b the introducer needle 15a is fixed to the threaded hub 15c of the introducer needle 15a. The threaded hub 15c comprises an outer thread mating an inner thread of the threaded sleeve 15b. The pitch of the threaded connection in the introducer cap 15 and the pitch of the threaded connection in the base 10 is the same, which means that a rotation of 360° of the hub 13 and the hub 15c result in identical translations. In order to transfer a rotation of the hub 13 to the hub 15c, hub 13 comprises a coupling end 13c and the hub 15 comprises a coupling 15d, see e.g. figure 7b. When the introducer cap 15 is attached to the base 10 hub 13 and hub 15c are coupled rotationally as shown in figure 5. If a using person rotates the protective sleeve 16 rotation is transmitted to the hub 13 and further transmitted to the hub 15c such the infusion cannula 12 and the introducer needle move in unison.

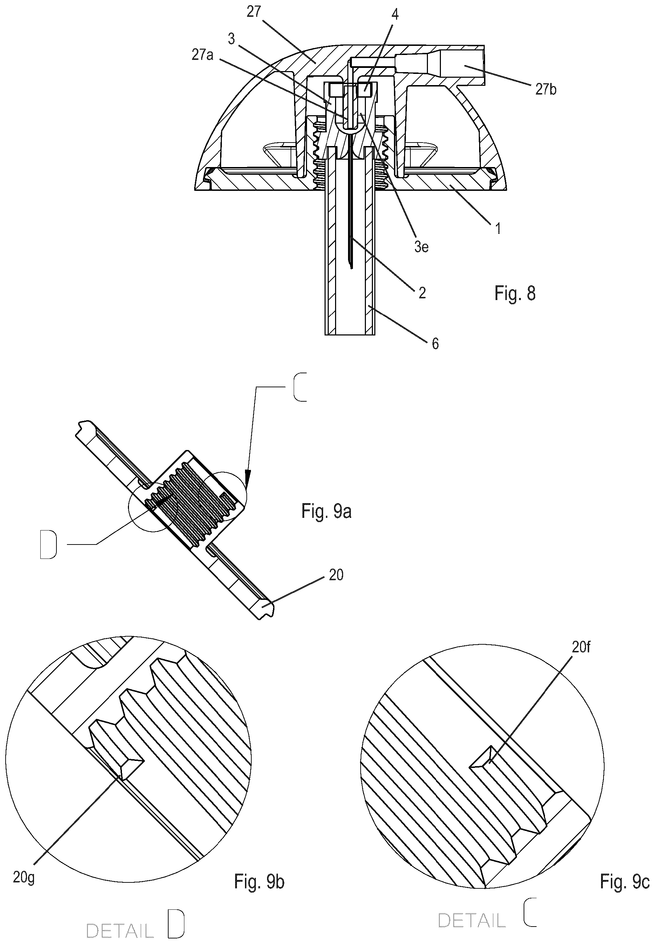

Figure 8 shows the first embodiment with base 1 but advanced with an attached infusion cap 27. The infusion cap 27 may also be applied to any of the bases shown in present document. It comprises the connection cannula 27a which pierces the septum, as shown in figure 6. It further comprises the opening 27b for the infusion tube. The actual infusion tube as well as the connector for connecting the infusion tube are not shown for the sake of clarity, but are obvious to any person skilled in the art. Opening 27b and connection cannula 27a are in fluid connection such that infusion fluid from an infusion tube may flow from the opening 27b through the connection cannula 27a into the cavity 3e and further through the infusion cannula 2 into the tissue of the using person.

Figures 9a to 10c show details regarding movement constraints of the infusion cannula hub in the opening of the base. Basically, the variants may be used with all bases disclosed in this document. For reasons of conciseness, the movement constraints are discussed into separate examples. Figures 9a to 9c show a first possibility to constrain movement of the infusion cannula hub between a lower and an upper end position. In this example movement is stopped by the radial stops 20f and 20g (see figure 9b and 9c for magnified details) which block rotation of the infusion cannula hub (e. g. hub 3 or hub 13) beyond their positions. Ideally but not necessarily the thread on the infusion cannula hub comprises geometrically well-defined ends or end surfaces to provoke an immediate and well perceivable stop in motion as one of the radial stops 20f or 20g is reached.

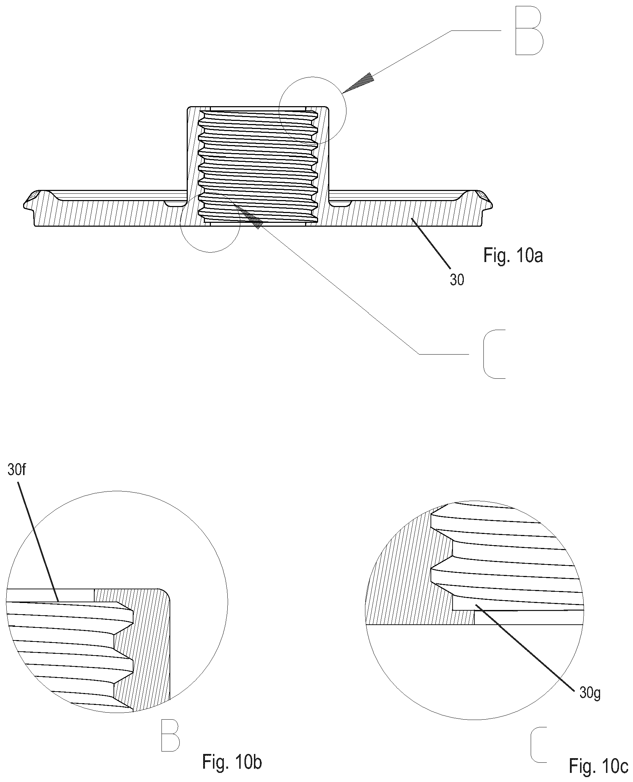

An alternative for movement constraints is shown in figures 10a to 10c. Here the movement constraints are realized by axial limitations, namely the axial stops (or stop surfaces) 30f and 30g.

Movement constraint as shown figures 9a or 10a may also be combined or partly combined without a deviation from the inventive idea.

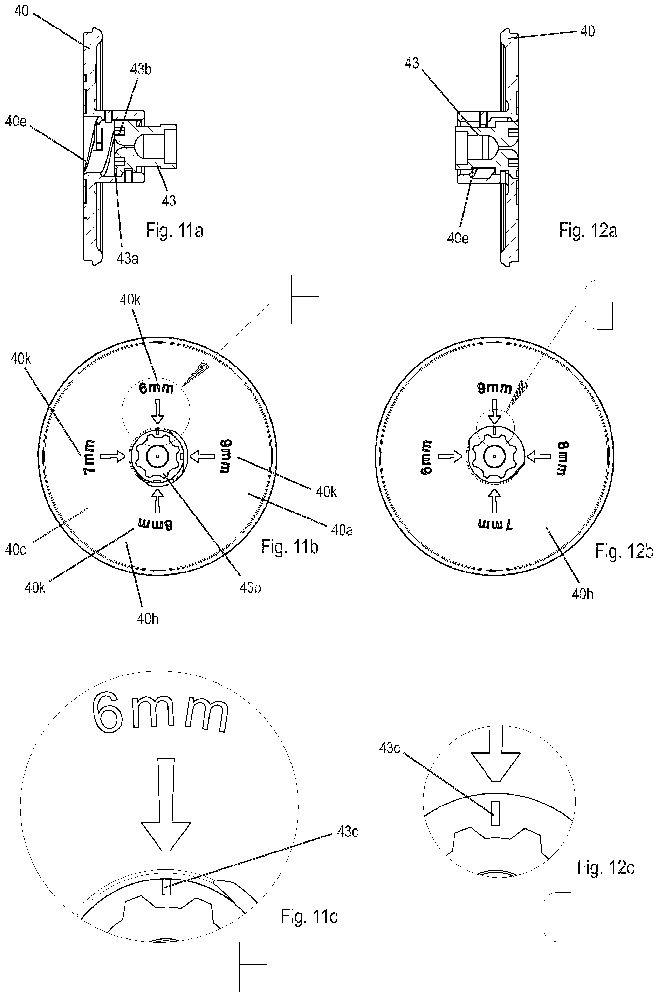

Figures 11a to 12c show a possible embodiment of insertion depth markings also applicable (with obvious adaptions) to the embodiments described above and below. On the lower side 40a the is the adhesive patch 40 arrangement, however, not visible as the adhesive patch 40c is covered by liner 40. Liner 40h covers the adhesive surface of the adhesive patch 40c which in use of the infusion set will be attached to the skin of the using person, therefore, the liner 40h will be removed from the adhesive patch 40c prior to attachment to the skin. Figure 11b shows view onto the lower side 40a of the base 40 with liner 40h. On the liner 40h, four insertion depth markers 40k with an corresponding number for the insertion depth and an arrow to mark a position are arranged. Infusion cannula hub 43 comprised a marking 43c which indicates the rotational position of the infusion cannula hub 43. When a tip of one of the arrows align with the marking 43c the number related the arrow denotes the set insertion depth for the infusion cannula (not shown) attached to the infusion cannula hub 43. Pitch of threaded connection 40e/43a have of course to match the insertion depth markers 40k and the marking 43c. Figures 11a to 11c show an insertion depth of the 6 mm, where the infusion cannula hub 43 is on its rightmost position (as may be seen in figure 11a) corresponding to minimum insertion depth. Figures 12a to the 12c show the position of the infusion cannula hub 43 corresponding to the maximum insertion depth of 9 mm (ref. figures 12b and 12c).

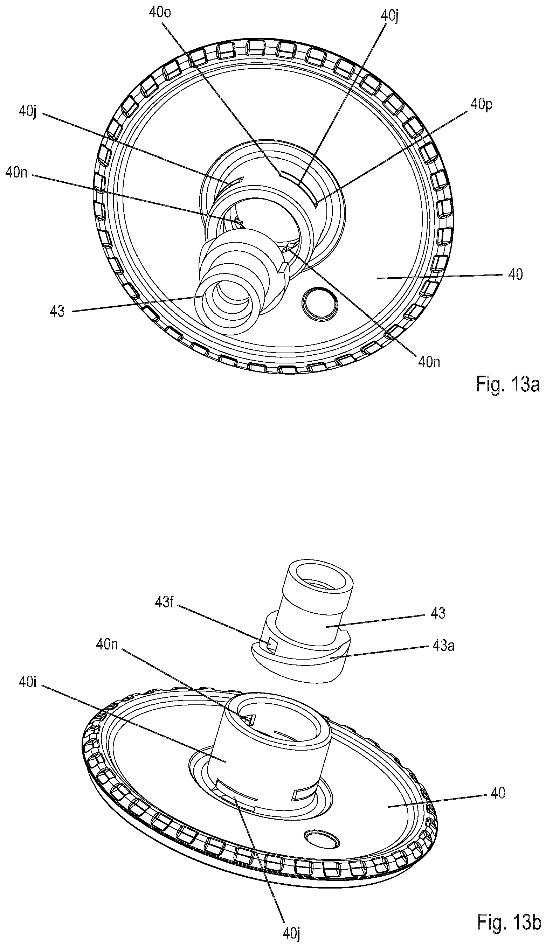

Figures 13a and 13b show an additional functional improvement, here applied the embodiment of figures 11a to 12c. It would also be applicable to other embodiments disclosed herein. For the convenience of use it may make sense to have defined rotational positions at which the rotation is stopped and the position is kept. This may be achieved by a latching mechanism similar to a ratchet or realized in form of at least one ratchet. For example in the last embodiment there were 4 marked insertion depths and it makes sense to stop the rotation of the infusion cannula hub 43 at these predefined positions. Such a possible mechanism is shown in figures 13a and 13b. Wall 40i of base 40 comprises for flexible arms 40j with one end 40o and a free flexible end 40p. On the free end 40p there is a tooth 40n arranged directed to the thread of the infusion cannula hub 43. The surface of the thread 43a flexes the free end 40p outwardly. On the surface of the thread 43a (or more generally on the hub 43) a dent 43f is arranged. Depending on the rotation of the infusion cannula hub 43 a tooth 40n may hit the dent 43f and consequently the free end 40 may flex inwards such that the tooth 40 may enter the dent 43f. In order further rotate the infusion hub 43 one has to overcome the resistance imposed by the tooth and free end to be flexed out of the dent 43f again.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 1: base
- 1a: lower side
- 1b: upper side
- 1c: adhesive patch
- 1d: opening
- 1e: inner thread of the opening
- 1i: wall
- 1m: rim
- 2: infusion cannula
- 2a: maximum insertion depth
- 2b: minimum insertion depth
- 2c: tip of infusion cannula
- 3: hub of infusion cannula
- 3a: thread on the outer surface of the thread
- 3b: coupling groove
- 3e: lumen
- 4: septum
- 5: introducer cap
- 5e: flexible part
- 5f: coupling teeth
- 5h: retaining wing
- 5i: retaining tab
- 6: protective sleeve
- 6a: longitudinal ribs

- 10: base
- 10a: lower side
- 10b: upper side
- 10c: adhesive patch
- 10d: opening
- 10e: inner thread of the opening

- 12: infusion cannula
- 12c: tip of infusion cannula
- 13: hub of infusion cannula
- 13a: thread on the outer surface of the thread
- 13b: coupling groove
- 13c: coupling end
- 14: septum
- 15: introducer cap
- 15a: introducer needle
- 15b: threaded sleeve
- 15c: threaded hub of introducer needle (threaded end)
- 15d: coupling
- 15h: retaining wing
- 15i: retaining tab
- 16: protective sleeve
- 20: base
- 20f: radial stop
- 20g: radial stop
- 27: infusion cap
- 27a: connection or cap cannula
- 27b: opening for infusion tube
- 30: base
- 30f: axial stop
- 30g: axial stop
- 40: base
- 40a: lower side
- 40c: adhesive patch (not visible)
- 40e: inner thread
- 40h: liner
- 40i: wall
- 40j: flexible arm
- 40k: insertion depth markers
- 40n: tooth
- 40o: fixed end
- 40p: free end
- 43: hub of the infusion needle
- 43a: outer thread on hub
- 43b: coupling groove
- 43c: marking
- 43f: dent

## Claims

1. An infusion set comprising:
a base (1) having upper (1b) and lower (1a) sides,
an adhesive patch (1c) mounted on the lower side (1a) of the base (1),
an opening (1d) in the base (1) between the upper (1b) and the lower (1a) side of the base, and
an infusion cannula (2) having a lumen and a longitudinal axis,
the infusion cannula (2) extending downwardly from the lower side (1a) of the base and having a open distal end to be inserted into a patients tissue,
the infusion cannula (2) further having a proximal end with a hub (3) which is fixedly attached to the infusion cannula (2),
the hub (3) having a fluid passage for allowing fluid to pass into the lumen of the infusion cannula (2),
**characterized in that**
the infusion set further comprising a protective sleeve (6) removably attached to the hub (3) and covering the infusion cannula (2), wherein the protective sleeve (6) is rotationally fixed to the hub (3),
the hub (3) is being disposed moveably in the opening (1d) of the base (1) and the hub (3) having a threaded outer surface (3) being in a threaded connection with a threaded inner surface (1e) of the opening (1d),
the hub (3) being moveable between a lower position and an upper position within the opening (1d) by rotating the protective sleeve (6) around the longitudinal axis of the infusion cannula (2) thereby setting a length of the infusion cannula (2) extending from the lower side (1a) of the base (1).

2. The infusion set according to claim 1, wherein the threaded inner surface (20e) of the opening (20d) comprises a stop surface (20f) at the lower end of the threaded surface (20e) and a further stop surface (20g) at the upper end of the threaded surface (20e) for limiting the movement of the hub (20).

3. The infusion set according to claim 2, wherein the stop surface (20f) and the further stop surface (20g) are oriented approximately parallel to the upper side of the base (20).

4. The infusion set according to claim 2, wherein the stop surface (30f) and the further stop surface (30g) are oriented roughly perpendicular to the upper side of the base (30).

5. The infusion set according to any of the preceding claims, wherein the fluid passage in the hub (3) is closed by a pierceable septum (4).

6. The infusion set according to any of the preceding claims, wherein the fluid passage in the hub (3) comprising funnel beneath the septum (4).

7. The infusion set according to any of the preceding claims, the infusion set further comprising an infusion cap (27) to be rotatably and removably attachable to the upper side of the base (1), the infusion cap comprising a cap cannula (27a) for piercing the pierceable septum (4).

8. The infusion set according to the preceding claim, wherein the infusion cap (27) being fixedly connected to an infusion tube which is in fluid connection with the cap cannula (27a).

9. The infusion set according to claim 5, wherein the infusion cannula (12) is made from a polymeric material and an introducer cap (15) adapted to be pressed against the upper side of the base (10) and adapted to be removably attached to the base (10), the introducer cap (15) comprising upper and lower sides, and an introducer needle (15a) extending downwardly from the lower side, the introducer needle (15a) being adapted to extend through the pierceable septum (14) and through the infusion cannula (12) on the lower side of the base (10).

10. The infusion set according to the preceding claim, wherein at least one retaining wing (15h) and one retaining tab (15i) are arranged on the upper side of the introducer, which are configured to be at least partly and releasably inserted into an insertion device for the infusion set, wherein the insertion device comprises at least one retaining element for retaining the infusion set through the introducer cap (15).

11. The infusion set according to claim 9, wherein the introducer needle (15a) is fixedly and non-moveably extending from the lower side of the introducer cap (15), and wherein during movement of the hub (13), the infusion cannula (12) moves along the longitudinal axis relative to the introducer needle (15a).

12. The infusion set according to claim 9, wherein the introducer needle (15a) disposed moveably on the lower side of the introducer cap(15),
wherein a threaded sleeve (15b) is disposed in the center of the lower side of the introducer cap (15),
wherein the end of the introducer needle (15a) pointing towards the introducer cap (15) comprises a threaded end (15c) in threaded connection with threaded sleeve (15b),
wherein the pitch of the threaded connection between the threaded sleeve (15b) and the threaded end (15c) is the same as the pitch of the threaded connection between the thread of the threaded outer surface of the hub (13) and the treaded inner surface (10e) of the opening in the base.

13. The infusion set according to claim 12, wherein when the introducer cap (15) is attached to the base (10), the introducer needle (15a) and the hub (13) rotate and translate in unison.

14. The infusion set according to claim 5, wherein the infusion cannula (2) is made from steel.

15. The infusion set according to any of the preceding claims, wherein either threaded surface of the opening (40e) or the threads (43a) on the hub (43) comprise ratchet teeth or a ratchet dent (43f) and the other comprises at least one flexible element (40jwith at least one counter tooth (40n) to be in mechanical contact with the ratchet teeth.

16. The infusion set according to any of the preceding claims, wherein the hub (3) being moveable between a lower position and an upper position for a distance of at least 1 mm, preferably at least 3 mm.
